# FASCICULE DE BREVET EUROPEEN

(11) **EP 0 763 111 B1**
(45) Date de publication et mention de la délivrance du brevet: **09.11.2005**
(21) Numéro de dépôt: 95923370.1
(22) Date de dépôt: 12.06.1995
(51) Int. Cl.: C12N 15/17, C12N 15/85, C12N 5/10, C12N 1/21, C07K 14/62, C07K 16/26, A61K 38/28, A61K 39/395, G01N 33/563, G01N 33/74

(54) **EPIL/PLACENTINE**
EPIL/PLAZENTIN
EPIL/PLACENTINE

(30) Priorité: 13.06.1994 FR 9407191
(43) Date de publication de la demande: 19.03.1997
(73) Titulaire: UNIVERSITE RENE DESCARTES PARIS V, 75270 Paris (FR)
(72) Inventeur: KOMAN, Ahmet, F-78240 Chambourcy (FR); CHASSIN, Dorine, F-94300 Vincennes (FR); BELLET, Dominique, F-92170 Vanves (FR)
(74) Mandataire: Warcoin, Jacques
(86) Numéro de dépôt international: PCT/FR1995/000766
(87) Numéro de publication internationale: WO 1995/034653

(56) Documents cités:
- DATABASE WPI Week 7338 Derwent Publications Ltd., London, GB; AN 56444u & JP,B,48 030 370 (SANSEI SEIYAKU CO LTD)
- DATABASE WPI Week 7119 Derwent Publications Ltd., London, GB; AN 32716s & SU,A,273 369 (LITVINOV VV)
- DATABASE BIOSIS BIOSCIENCES INFORMATION SERVICE, PHILADELPHIA, PA, US AN 70:132499, LITVINOV V.V 'Active preparation from human placental tissue' & VRACH DELO, vol. 5, 1969 pages 120-122, LITVINOV VV
- CHEMICAL ABSTRACTS, vol. 78, no. 22, 4 Juin 1973 Columbus, Ohio, US; abstract no. 140430a, ALTUNYAN ET AL. 'Amino acid composition of a new blood substituted from the placenta, aminoplacentin' page 281; colonne 140435; & BIOL. ZH. ARM., vol. 8, 1972 pages 99-100, ALTUYAN ET AL.

### LISTE DE SEQUENCES

(1) INFORMATIONS GENERALES:
   (i) DEPOSANT:
      (A) NOM: INSTITUT GUSTAVE-ROUSSY
      (B) RUE: 39 RUE CAMILLE DESMOULINS
      (C) VILLE: VILLEJUIF
      (E) PAYS: FRANCE
      (F) CODE POSTAL: 94800
   (ii) TITRE DE L' INVENTION: NOUVELLE PROTEINE DENOMMEE EPIL/PLACENTIN, PROCEDE DE PREPARATION DE CETTE PROTEINE ET COMPOSITION PHARMACEUTIQUE LA CONTENANT, ADN CODANT POUR LADITE PROTEINE
   (iii) NOMBRE DE SEQUENCES: 1
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 618 paires de bases
      (B) TYPE: nucléotide
      (C) NOMBRE DE BRINS: simple
      (D) CONFIGURATION: linéaire
   (ii) TYPE DE MOLECULE: ADNc
   (ix) CARACTERISTIQUE:
      (A) NOM/CLE: CDS
      (B) EMPLACEMENT:107..523
   (xi) DESCRIPTION DE LA SEQUENCE: SEQ ID NO: 1:
   CAGTCTGGAG CCCAGAAGGG ACACACCAGC ACAGTCTGGT AGGCTACAGC AGCAAGTCTC       60
   TAAAGAAAGG CTGAGAACAC CCAGAACAGG AGAGTTCAGG TCCAGG ATG GCC AGC         115
                                                      Met Ala Ser
                                                        1
   CTG TTC CGG TCC TAT CTG CCA GCA ATC TGG CTG CTG CTG AGC CAA CTC        163
   Leu Phe Arg Ser Tyr Leu Pro Ala Ile Trp Leu Leu Leu Ser Gln Leu
         5                  10                  15
   CTT AGA GAA AGC CTA GCA GCA GAG CTG AGG GGA TGT GGT CCC CGA TTT        211
   Leu Arg Glu Ser Leu Ala Ala Glu Leu Arg Gly Cys Gly Pro Arg Phe
    20                  25                  30                  35
   GGA AAA CAC TTG CTG TCA TAT TGC CCC ATG CCT GAG AAG ACA TTC ACC        259
   Gly Lys His Leu Leu Ser Tyr Cys Pro Met Pro Glu Lys Thr Phe Thr
                    40                  45                  50
   ACC ACC CCA GGA GGG TGG CTG CTG GAA TCT GGA CGT CCC AAA GAA ATG      307
   Thr Thr Pro Gly Gly Trp Leu Leu Glu Ser Gly Arg Pro Lys Glu Met
               55                  60                  65
   GTG TCA ACC TCC AAC AAC AAA GAT GGA CAA GCC TTA GGT ACG ACA TCA      355
   Val Ser Thr Ser Asn Asn Lys Asp Gly Gln Ala Leu Gly Thr Thr Ser
           70                  75                  80
   GAA TTC ATT CCT AAT TTG TCA CCA GAG CTG AAG AAA CCA CTG TCT GAA      403
   Glu Phe Ile Pro Asn Leu Ser Pro Glu Leu Lys Lys Pro Leu Ser Glu
       85                  90                  95
   GGG CAG CCA TCA TTG AAG AAA ATA ATA CTT TCC CGC AAA AAG AGA AGT      451
   Gly Gln Pro Ser Leu Lys Lys Ile Ile Leu Ser Arg Lys Lys Arg Ser
   100                 105                 110                 115
   GGA CGT CAC AGA TTT GAT CCA TTC TGT TGT GAA GTA ATT TGT GAC GAT      499
   Gly Arg His Arg Phe Asp Pro Phe Cys Cys Glu Val Ile Cys Asp Asp
                   120                 125                 130
   GGA ACT TCA GTT AAA TTA TGT ACA TAGTAGAGTA ATCATGGACT GGACATCTCA     553
   Gly Thr Ser Val Lys Leu Cys Thr
               135
   TCCATTCTCA TATGTATTCT CAATGACAAA TTCACTGATG CCCAATTAAA TGATTGCTGT    613
   TTAAA                                                                618

## Description

La présente invention concerne une nouvelle protéine dénommée placentine ou EPIL, ses analogues, leurs procédés de préparation ainsi que leurs applications.

L'insuline, l'IGF-1, l'IGF-2 et la relaxine appartiennent à une famille d'hormones peptidiques ayant certaines structures et fonctions en commun, notamment leur influence sur la prolifération, le développement, la différenciation et le métabolisme cellulaire.

L'insuline est bien connue comme étant une hormone endocrine pancréatique régulant le métabolisme énergétique. Les facteurs de croissance de type insuline-IGF-1 sont des peptides promoteurs de croissance impliqués dans la régulation endocrine, paracrine et autocrine de la croissance cellulaire et qui sont exprimés dans de nombreux tissus. L'IGF-2 a des propriétés similaires mais est exprimée en quantité plus importante en période prénatale et est considérée comme étant un facteur de croissance foetale.

La relaxine induit un remodelage des tissus conjonctifs dans le tractus reproductif et inhibe les contractions utérines. Son rôle fonctionnel dans le cerveau où une expression très importante a été obtenue reste à élucider.

On a adjoint, récemment, à cette famille les Ley I-L qui sont actuellement clonés sous forme d'ADNc et dont l'activité biologique est encore à définir. Cette famille peptidique présente en commun des caractères structuraux définis par la position de différentes cystéines essentielles pour la formation d'une structure tertiaire.

L'insuline qui est le prototype de cette famille comprend deux chaînes peptidiques A et B reliées par des ponts disulfure. Elle est codée par un ARNm qui est traduit en pre-pro insuline. Le peptide signal, de même que le peptide de jonction C, sont excisés par modification post-traductionnelle ; ceci est également valable pour la relaxine alors que les IGFs sont maturés différemment sans élimination du peptide C et demeurent sous forme de chaîne unique. On a pu démontrer que tous les membres de cette famille se fixaient à des récepteurs de surface cellulaires. Ces récepteurs ont été identifiés par clonage moléculaire et caractérisés en détail pour l'insuline et l'IGF. Ils appartiennent à la super-famille des récepteurs tyrosine-kinases (RTK's) qui comprend des récepteurs de facteurs de croissance et leurs analogues oncogènes tels que c-erbB2/neu (récepteur EGF), c-met (récepteur du facteur de croissance des hépatocytes), fms (récepteur de CSF-1) et trk (récepteur de NGF).

La voie de transduction du signal intracellulaire pour ces récepteurs est caractérisée par une activité tyrosine-kinase qui produit une autophosphorylation des résidus tyrosine sur le récepteur suivie par une chaîne d'évènements correspondant à des phosphorylations. Ceci inclut notamment l'activation de l'IRS-1 (en particulier pour l'insuline et l'IGF), PI3K, Shc, GBR2, Sos, Ras, Raf et la protéine activant la mitogénèse (MAP) kinase notamment lorsque la cascade de phosphorylation affecte différents processus cellulaires tels que la transcription.

Les effets physiologiques pléiotropiques de cette cascade de signaux en général font l'objet de recherches intensives.

La présente invention concerne plus particulièrement une nouvelle molécule de cette famille de l'insuline qui est dénommée ci-après placentine ou EPIL (Early Placenta lnsulin Like peptide) et dont la séquence d'acides aminés et celle de l'ADN qui code pour cette protéine correspond à la séquence ID 1.

La placentine ou EPIL a été isolée à partir d'une banque d'ADNc de cellules cytotrophoblastiques préparées à partir de placenta prélevé lors du premier trimestre de la grossesse. L'analyse par Northern blot (effectuée sur un très grand échantillonnage de tissus normaux) a révélé des niveaux d'ARN détectables seulement dans le tissu placentaire.

La séquence d'acides aminés qui a été obtenue montre, pour la protéine selon l'invention, un arrangement de résidus cystéine caractéristique de la famille de l'insuline.

Le traitement de cellules cibles avec des milieux conditionnés à partir de cellules cultivées exprimant la protéine recombinante parait induire un schéma de phosphorylation de la tyrosine similaire à celui observé après traitement avec l'insuline.

Enfin, des expériences préliminaires suggèrent que l'EPIL/placentine induit la synthèse d'ADN.

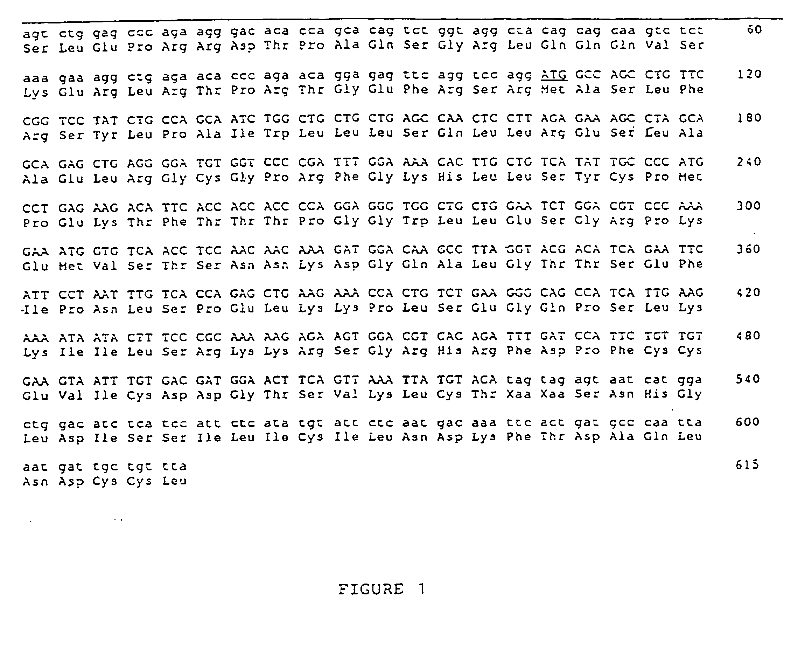

C'est pourquoi la présente invention concerne plus particulièrement une protéine dénommée EPIL/placentine, protéine de formule correspondant à la séquence ID 1, représentée également figure 1, et ses analogues, présentant une activité de type EPIL/placentine et obtenue par délétion et/ou substitution.

Sur la séquence ID 1, la structure de l'EPIL/placentine correspond à la structure en acides aminés suivant la méthionine en position 36 jusqu'à l'acide aminé en position 174.

Les références aux séquences d'ADN de l'EPIL/placentine correspondent à cette séquence.

Les séquences nucléiques présentes avant le premier ATG présumé (souligné) et après le codon stop sont en minuscule.

Les analogues de lEPIL/placentine sont les protéines ou peptides qui présentent une forte homologie, en particulier plus de 60 % d'homologie, de préférence 80 %, en acides aminés avec le composé correspondant à la séquence ID 1 et qui ont pu être obtenus par délétion ou par substitution tout en conservant les caractéristiques essentielles de l'EPIL/placentine. Ces analogues seront parfois nommés "composés de type EPIL/placentine".

En particulier, on peut citer des fragments qui présentent certains des épitopes caractéristiques de l'activité de l'EPIL/placentine, notamment les protéines de type EPIL/placentine qui présentent au moins les 20 premiers acides aminés de l'extrémité N-terminale de la séquence ID 1.

La présente invention concerne bien entendu l'EPIL/placentine ou ses analogues sous forme glycosylée ou non glycosylée. On peut citer également les protéines présentant ou non les ponts disulfures de la protéine d'origine. En effet, cette protéine peut être préparée par extraction à partir d'échantillons biologiques mais sera de préférence obtenue par des techniques mettant en oeuvre le génie génétique et dont les structures secondaires pourront varier en fonction de l'organisme hôte.

L'invention concerne également une molécule choisie parmi :
(a) la séquence d'ADN de la séquence ID 1, et
(c) les séquences d'ADN qui compte tenu de la dégénerescence du code génétique correspondent aux séquences (a) ou (b) et qui codent pour un polypeptide de type EPIL/placentine ou ses analogues selon l'invention.

En particulier, l'invention concerne la séquence d'ADN correspondant à la séquence ID 1.

Bien entendu, les séquences d'ADN mentionnées précédemment peuvent être des séquences génomiques ou de type génomique, c'est-à-dire que certains des éléments peuvent être séparés par des introns qui seront excisés pour conduire à l'expression de l'EPIL/placentine mature.

Les séquences d'ADN précédentes pourront être incorporées dans des vecteurs de elonage ou d'expression de l'EPIL/placentine ou de ses analogues, sous le contrôle d'éléments assurant leur expression dans une cellule hôte définie, ces secteurs faisant partie de l'invention.

Les systèmes d'expression dans les cellules procaryotes ou eucaryotes sont bien connus de l'homme de métier, il pourra s'agir notamment de systèmes de type plasmidique ou de type viral comportant des promoteurs assurant l'expression dans la cellule hôte et munis également des éléments terminateurs nécessaires.

Mais il pourra également s'agir de vecteurs d'intégration qui pourront comporter leur propre système d'expression ou bien être conçus pour s'intégrer dans les chromosomes à des endroits où ils se trouveront sous la promotion d'un promoteur chromosomique, notamment dans le cas des cellules procaryotes, en utilisant la technique des recombinaisons homologues.

La présente invention concerne également des cellules hôtes caractérisées en ce qu'elles comportent un vecteur autoréplicatif exprimant l'EPIL/placentine ou un analogue de l'EPIL/placentine selon l'invention.

Comme cela a été indiqué précédemment, il pourra s'agir de cellules eucaryotes, en particulier de cellules de mammifères type cellule CHO, ou de cellules en culture d'autres types plus appropriées à la préparation de l'EPIL/placentine sous sa forme mature ; mais il est également possible d'envisager l'utilisation de cellules bactériennes par exemple : dans ce cas il pourra être utile de faire subir des remaniements complémentaires à la protéine ainsi obtenue.

Il s'agit là encore de techniques qui sont connues de l'homme de métier et qui ne seront pas décrites plus complètement, sauf dans le cadre de certains des exemples ci-après.

Les cellules ainsi transformées par un vecteur autoréplicatif exprimant la protéine pourront être utilisées par culture dans un procédé permettant de préparer l'EPIL/placentine ou ses analogues.

Il s'agira notamment d'un procédé de préparation de l'EPIL/placentine ou ses analogues caractérisé par la mise en culture de cellules dont on extraira directement ou à partir du milieu de culture l'EPIL/placentine ou ses analogues.

L'EPIL/placentine pourra notamment être extraite par immuno-purification.

La présente invention concerne également les produits de recombinaison obtenus par le procédé mis en oeuvre précédemment.

Dans certains cas, la protéine pourra être préparée sous forme fusionnée, en particulier lorsqu'il s'agira d'un analogue de l'EPIL/placentine de type peptidique, ou bien lorsque la fusion avec cette protéine permettra d'atteindre plus facilement le site d'action de l'EPIL/placentine ou de ses analogues, ou lorsque, dans certains cas, cette protéine permettra de leurrer certains systèmes naturels en assurant une plus longue durée de vie de la protéine en cause.

La présente invention concerne enfin des compositions pharmaceutiques utilisant l'EPIL/placentine ou ses analogues à titre de principe actif.

Comme cela a été indiqué, l'EPIL/placentine peut présenter un grand nombre d'activités, en particulier elle peut présenter comme d'autres facteurs humains de croissance de type 1 une activité au niveau cardiaque, notamment dans le traitement et la prévention de certains troubles cardiaques tels que les insuffisances cardiaques aiguës.

Cette protéine ou certains de ses analogues peuvent présenter une activité de type facteur de croissance et promoteur de la lactation. Egalement, les produits peuvent être utilisés dans le processus de régénération de tissus nerveux, musculaire, cutané ou osseux, notamment en cas de pathologies dégénératives ou endocriniennes, de lésions traumatiques ou d'affections virales.

Enfin, l'EPIL/placentine ou ses analogues peuvent présenter une action au niveau du contrôle de l'ensemble des phénomènes de la conception chez l'homme ou chez les animaux.

La présente invention concerne également des anticorps et plus spécialement des anticorps monoclonaux spécifiques de cette protéine ou de ses analogues ainsi qu'une méthode de diagnostic in vitro permettant, à l'aide de l'EPIL/placentine ou de ses analogues et/ou des anticorps correspondants, de détecter des taux anormaux d'EPIL/placentine dans les échantillons physiologiques ou non.

Des fragments d'ADN correspondant aux séquences mentionnées précédemment pourront être intégrés dans une stratégie thérapeutique "sens" ou "anti-sens" ; quant à l'EPIL/placentine ou ses analogues, ils pourront également être intégrés dans une stratégie de ciblage de certaines molécules pharmaceutiquement actives vers les récepteurs de l'EPIL/placentine ou ses analogues.

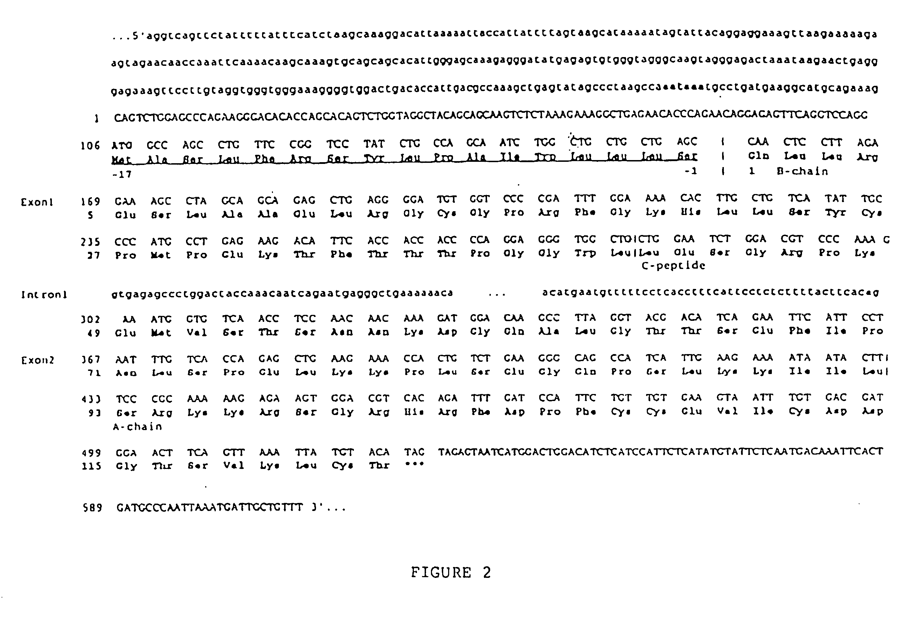

La structure détaillée de l'EPIL/placentine correspond à la figure 2, c'est-à-dire un peptide signal position -17 à -1, une chaine B 1 à 41, un peptide de connexion C de 42 à 92 puis une chaine A de 93 à 122.

Cette structure apparente l'EPIL/placentine à la pro-insuline ou à la prorelaxine.

En particulier, la position des ponts disulfures doit conduire à une structure tridimensionnelle du type de l'insuline.

D'autres caractéristiques et avantages de la présente invention apparaitront à la lecture des exemples ci-après qui sera effectuée en se référant aux séquences ID 1 et figure 2 correspondant à la séquence de l'EPIL/placentine et du gène humain codant pour cette protéine.

### EXEMPLES

La mise en évidence de l'EPIL/placentine est le fruit d'une recherche spécifique tendant à mettre en évidence les molécules impliquées dans la croissance cellulaire et/ou dans les processus néoplasiques.

Bien que le placenta soit un tissu normal, les cellules constituantes présentent de nombreuses propriétés communes avec les cellules néoplasiques, notamment les cytotrophoblastes invasifs et hautement mitotiques qui peuvent pénétrer le tissu maternel. Ces trophoblastes sont une source de facteurs de croissance, d'hormones et de récepteurs de facteurs de croissance notamment.

Les études actuelles montrent que les gènes préférentiellement exprimés dans les cellules trophoblastiques peuvent aussi être exprimés préférentiellement dans les cellules néoplasiques.

Mais tenant compte du fait que le placenta, et notamment les trophoblastes, demeurent sous un contrôle complet durant la grossesse normale, les molécules trophoblastiques correspondant à ce contrôle sont des candidats très intéressants à titre d'agent anticancéreux ou susceptibles de contrôler le processus de cancérisation.

C'est pourquoi la méthode d'isolement qui a été utilisée est une méthode de soustraction de ADNc mettant en oeuvre la technologie PCR sur des trophoblastes jeunes. Ceci a permis de mettre en évidence des gènes qui sont surexprimés dans ces cellules et qui pourraient avoir un rôle dans la croissance cellulaire et dans la régulation de cette croissance.

### MATERIEL ET METHODES

### Tissus

Des placentas de 5 à 12 semaines et des placentas à terme, de même que des échantillons chirurgicaux de tissus normaux et tumoraux, ont été réfrigérés immédiatement (dans les 10 minutes suivant l'intervention chirurgicale) et conservés dans l'azote liquide jusqu'à la préparation des ARN. Ces collections de tissus ont été obtenues et utilisées en accord avec les protocoles approuvés par les Comités des différents hôpitaux.

### Cellules

Les lignées de cellules humaines utilisées pendant cette étude et leurs origines histologiques sont les suivantes : choriocarcinome gestationnel (JAr et JEG-3) ; carcinome hépatocellulaire (PLC/PRF/5 et Hep G2) ; adénocarcinome du colon (LS180) ; carcinome de l'ovaire (OV1/p, OV1VCR), la lignée de cellules OV1/VCR est dérivée de OV1/p et est résistante à la vincristine ; carcinome épidermoïde (A431) ; carcinome du poumon (A427) ; carcinome épithéloïde du cervix (HeLa) ; carcinome mammaire (McF7, MDA-MB-361, SK-BR-3, BT-20 et BT-474) ; cellules mammaires transformées par SV-40 (HBL-100) ; lignée cellulaire de neuroblastome (SHSY-5Y) ; lignée cellulaire de fibroblaste normal (CCL-137).

Toutes ces lignées cellulaires étant disponibles à l'ATCC à l'exception de l'IGR/OV1 (OV1/p) et d'OV1/VCR.

Ces lignées cellulaires sont mises en croissance dans un milieu DMEM ou RPMI-1460 (Gibco-BRL Laboratoires, Gaithersburg, MA) supplémenté avec 10 % de sérum de veau foetal inactivé par la chaleur, 10 µM d'acides aminés non essentiels, 4 mM de glutamine, 100 U/ml de pénicilline et 100 µg/ml de streptomycine à 37°C avec 5 % de CO2.

### Isolement des cytotrophoblastes

Les cytotrophoblastes sont purifiés comme cela est décrit par Kliman et al. Endocrinology, 118: 1567-1582, 1986. En bref, les tissus villeux du placenta du premier trimestre sont dispersés avec de la trypsine et de la déoxyribonucléase 1. Les cellules dispersées sont alors purifiées sur un gradient Percoll 5-70 % (Pharmacia). La bande à 1 040-1 060 g/ml de densité est collectée. L'examen microscopique révèle qu'elle comprend des cellules cytotrophoblastiques avec moins de 5 % de contamination par des cellules non trophoblastiques telles que les macrophages, les fibroblastes et les cellules endothéliales.

### Préparation de l'ARN

L'ARN total est préparé à partir de cultures de cellules préconfluentes ou de tissus réfrigérés en utilisant de l'isothiocyanate de guanidinine et par ultracentrifugation sur un gradient de chlorure de cesium en adaptant le protocole de Chirgwin et al. Les ARN des polysomes associés aux membranes du reticulum endoplasmique (MB-ARN) sont purifiés comme cela a été décrit précédemment. Après trypsination et purification sur gradient de Percoll, les cellules cytotrophoblastiques sont homogénéisées, puis les MB-ARN sont isolés sur gradient de sucrose en incluant un complexe ribonucléoside vanadyl comme inhibiteur de ribonucléase.

### Synthèse des ADNc et amplification par PCR

0,1 µg de MB-ARN est dissous dans 5 µl d'eau traitée DEPC et dénaturé avec 0,1 M MeHgOH et β-mercaptoéthanol. Le premier brin d'ADNc est synthétisé avec la transcriptase réverse du virus de la myoblastose aviaire (trousse Invitrogen, San Diego) en utilisant le primer-dT modifié indiqué ci-après (Frohman et al. Proc. Natl. Acad. Sci. USA, 85: 8998-9002, 1988). Les hétéroduplex ARN-ADNc de taille comprise entre 0,5 et 2 kb sont électroélués après migration dans un gel d'agarose à 2 %. Une queue d'oligo dG est ajoutée à l'extrémité 3' du premier brin d'ADNc avec la transférase déoxynucléotide terminale et l'ARN est éliminé par hydrolyse alcaline. Les ADNc sont amplifiés avec des amorces non spécifiques incluant les sites de restriction pour les enzymes NotI et SalI.

La séquence de l'amorce T utilisée pour la transcription réverse et la PCR est la suivante :

L'amorce C est identique à celle décrite dans Loh et al., (Science, 243 : 217-220, 1989) :

Le mélange réactionnel comporte 1,25 mM de chacun des 4 désoxyribonucléotides triphosphates, 0,5 µM de chaque amorce et 2,5 unités de Taq polymérase dans 50 mM KCl- 10 mM Tris-HCl (pH 8,3) - 3,5 mM MgCl₂-0,01 % gélatine. L'amplification est effectuée dans un thermocycleur pour 25 cycles de 20 secondes à 94°C, 30 secondes à 55°C et 1 minute à 72°C. Les produits sont chargés sur un gel d'agarose à 1 % à fusion basse. Puis la région 0,5-2 kb est excisée et réamplifiée dans les mêmes conditions. Les produits sont précipités, digérés totalement avec NotI et SalI, les tailles sont sélectionnées comme précédemment et électroéluées à partir d'agarose, précipitées et quantifiées.

L'ADNc total de placenta à terme et de lymphocytes T activés par de la phytohémaglutinine et cultivés pendant plusieurs jours en présence d'IL2 a été préparé en utilisant un système de synthèse d'ADNc double brin.

### Construction de la banque soustraite d'ADNc

L'hybridation soustractive est effectuée comme cela est décrit par Klickstein (Klickstein et al. Current protocols in molecular biology, pp 5.8.9.-5.8.15 Wiley Interscience, 1989). 0,2 µg d'ADNc de cellules cytotrophoblastiques amplifiés par PCR sont mis à digérer avec NotI et SalI, mélangés avec 8 µg d'ADNc de lymphocytes T activés et 8 µg d'ADNc de placenta à terme digérés avec AluI et RsaI, dissous dans 40 µl de tampon d'hybridation (50 % de formamide déionisé ; 10 mM de tampon phosphate sodium, pH 7 ; 5 X SSC ; 0,1 % SDS ; 10 mM EDTA), dénaturés 5 minutes à 98°C et incubés 24 heures à 37°C.

Les séquences communes entre cible et compétiteur sont reprises pour former des duplex entre les fragments courts (Alu/Rsa, compétiteur) et longs (Not/Sal de la cible), en inhibant la formation d'extrémités cohésives. Les brins d'ADNc complémentaires exprimés spécifiquement dans les cytotrophoblastes jeunes permettent la régénération des extrémités cohésives NotI et SalI pour un clonage unidirectionnel dans les sites pBSKII + phagemid de vecteur (Stratagene).

### Criblage de la banque

La banque d'ADNc soustraite est étalée sur milieu de culture gélosé et les colonies recombinantes sont prélevées et mises en culture dans le milieu LB puis amplifiées par PCR. Les ADN plasmidiques préparés par la méthode des minipréparations bouillies (Del Sal G. et al. Nucleic Acids Res., 16: 9878, 1988) et digérés avec NotI et SalI ou les produits des inserts amplifiés par PCR à l'aide des amorces originales sont analysés par Southern blot sur des gels à 1,2 % d'agarose. La taille moyenne des inserts est entre 500 et 1 000 nucléotides. Les gels sont transférés en double sur membranes de nylon (Hybond N, Amersham) dans un tampon alcalin. Les sondes utilisées pour l'hybridation sont des ADNc totaux synthétisés à partir des placentas jeunes, des placentas à terme et des lymphocytes T activés marqués au ³²P (Amersham) par multi-amorçage aléatoire. L'hybridation se déroule pendant 18 heures à 42°C suivie par un lavage stringent dans 0,1 X SSC à 50°C et la mise en autoradiographie.

### Analyse sur Northern blot

5 µg d'ARN total provenant de différents tissus et de lignées cellulaires sont analysés sur un gel à 1 % d'agarose contenant 2,2 M de formaldéhyde. Quand l'électrophorèse est terminée, le gel est rincé avec de l'eau bi-distillée et traité avec 10 x SSC pendant 30 minutes. L'ARN est alors transféré sur membrane de nitrocellulose renforcée (Schleicher et Schuell, Dassel) dans le tampon de transfert 20 X SSC pendant 18 heures. Les ARN transférés sont fixés sur les membranes par irradiation aux UV avant hybridation. Les sondes utilisées pour hybrider les membranes sont des inserts excisés marqués au ³²P par multi-amorçage aléatoire, ou des sondes monobrins générées par PCR en utilisant une amorce antisens universelle. L'hybridation est effectuée une nuit à 42°C suivie par des conditions de lavage stringentes dans 0,1 X SSC à 50°C et la mise en autoradiographie.

### Séquencage de l'ADN et analyse

Les ADN plasmidiques sont préparés par la méthode des minipréparations bouillies. Le séquençage de l'ADN est réalisé avec le kit Sequenase, version 2.0 (U.S. Biochemical). Les amorces sont soit des amorces universelles M13, T3,T7 ou des amorces spécifiques internes. Les produits de réaction sont analysés sur des gels contenant 6 % d'acrylamide et 50 % d'urée. Les séquences obtenues sont comparées aux séquences des différentes banques de données.

### Analyse

Les clones qui ne donnent pas de signal d'hybridation avec les sondes normalisées sont analysés par séquençage partiel et comparaison dans ces banques de données.

L'un des clones correspondant à l'EPIL/placentine correspond à la séquence en acides aminés correspondant à la séquence ID 1.

Les échantillons d'ARN totaux et polyA préparés à partir de lignées cellulaires transformées ou normales et des tissus correspondants sont analysés par Northern blot à l'aide de sondes ADNc d'EPIL/placentine antisens monobrin marquées. Les signaux d'hybridation sont détectés seulement dans les placentas, en particulier en début de grossesse, et sont pratiquement inexistants dans les autres tissus comme cela ressort du tableau ci-après (tableau I) :

**TABLEAU I**

| | |
|---|---|
| PLACENTA JEUNE | ++++ |
| PLACENTA A TERME | ++ |
| FOIE NORMAL | - |
| FOIE TUMORAL | - |
| VESSIE NORMALE | - |
| VESSIE TUMORALE | - |
| EPIPLOON NORMAL | - |
| EPIPLOON TUMORAL | - |
| OESOPHAGE NORMAL | - |
| OESOPHAGE TUMORAL | - |
| COLON NORMAL | - |
| COLON TUMORAL | - |
| ESTOMAC NORMAL | - |
| COL DE L'UTERUS TUMORAL | - |
| ENDOMETRE NORMAL | - |
| OVAIRE NORMAL | - |
| SEIN TUMORAL | - |
| GANGLION TUMORAL | - |
| RATE NORMALE | - |
| RECTUM NORMAL | - |
| RECTUM TUMORAL | - |
| TROMPE DE FALLOPE NORMALE | - |
| PEAU NORMALE | - |
| MYOMETRE NORMAL | - |
| GLANDE SURRENALE NORMALE | - |
| THYROIDE NORMALE | - |
| THYROIDE TUMORALE | - |

Des études effectuées sur des produits d'origine murine et simienne montrent des analogies indiquant des similitudes fonctionnelles significatives entre les espèces.

### Expression de l'EPIL/placentine

Pour l'expression eucaryotique de l'EPIL/placentine, l'ADNc a été inséré dans le vecteur d'eapression pBK-CMV dans l'orientation sens et antisens.

Deux types cellulaires ont été utilisés de façon à étudier les effets possibles des modifications post-traductionnelles inhérentes aux types cellulaires. Des cellules de rein de singe COS-7 ont été transfectées par DEAE pour l'expression transitoire tandis que des cellules trophoblastiques humaines transformées par SV40 (3AsubE) ont été transfectées par CaPO4 pour l'expression transitoire et stable.

Les cellules COS-7 et 3AsubE n'expriment pas de niveaux détectables d'ARNm d'EPIL/placentine en analyse par Northern blot. Les cellules transfectées sont conditionnées avec un milieu de culture exempt de sérum.

Les milieux conditionnés obtenus à partir des cellules transfectées sens ou antisens sont analysés pour tester la présence de protéines recombinantes.

L'activité biologique de la protéine recombinante est analysée sur différentes cellules cibles, considérant que les récepteurs spécifiques peuvent exister ou que la protéine peut se fixer sur d'autres récepteurs pour des molécules de structure proche, comme cela a été observé pour l'insuline et l'IGF.

Les résultats préliminaires observés suggèrent que l'EPIL/placentine induirait la tyrosine phosphorylation des protéines cellulaires et aurait une activité biologique sur la croissance des cellules trophoblastiques.

## Revendications

1. EPIL/placentine, protéine ayant la séquence ID 1, et ses analogues présentant une activité de type EPIL/placentine, obtenus par délétion et/ou substitution, **caractérisés en ce qu'**ils présentent as moins 60% d'homologie avec la séquence ID1.

2. EPIL/placentine selon la revendication 1, ayant la séquence ID 1.

3. EPIL/placentine ou analogues d'EPIL/placentine selon l'une des revendications 1 à 2, **caractérisés en ce que** la protéine est glycosylée.

4. Une molécule d'ADN choisie parmi :
(a) la séquence d'ADN ayant la séquence ID 1,
(b) les séquences d'ADN qui compte tenu de la dégénerescence du code génétique codent pour l'EPIL/placentine ou ses analogues selon la revendicaltion 1 ou 2.

5. Une molécule d'ADN ayant la séquence ID 1.

6. Vecteur d'expression exprimant l'EPIL/placentine ou un analogue de l'EPIL/placentine selon l'une des revendications 1 à 2, sous le contrôle d'éléments pouvant assurer son expression dans une cellule hôte.

7. Cellule hôte transformée, **caractérisée en ce qu'**elle comporte un vecteur autoréplicatif exprimant l'EPIL/placentine ou un analogue d'EPIL/placentine selon l'une des revendications 1 à 2.

8. Cellule selon la revendication 7, **caractérisée en ce qu'**il s'agit d'une cellule eucaryote.

9. Cellule selon la revendication 7, **caractérisée en ce qu'**il s'agit d'une bactérie.

10. Procédé de préparation d'EPIL/placentine ou d'un analogue de l'EPIL/placentine selon l'une, des revendications 1 à 2, **caractérisé par** la mise en culture de cellules selon l'une des revendications 7 à 9 dont on extraira directement ou à partir du milieu de culture l'EPIL/placentine ou ses analogues.

11. EPIL/placentine ou analogue d'EPIL/placentine obtenus par le procédé selon la revendication 10.

12. Composition pharmaceutique, **caractérisée en ce qu'**elle contient une EPIL/placentine ou un analogue d'EPIL/placentine selon l'une des revendications 1 à 3.

13. Anticorps monoclonaux dirigés contre l'EPIL/placentine ou un analogue d'EPIL/placentine selon l'une des revendications 1 à 3.

14. Méthode de diagnostic in vitro mettant en oeuvre l'EPIL/placentine ou un analogue de l'EPIL/placentine selon l'une des revendications 1 à 3 ou un anticorps monoclonal selon la revendication 13.

## Patentansprüche

1. EPIL/Placentin, Protein mit der Sequenz ID 1, und dessen Analoga, die eine Aktivität vom EPIL/Placentin-Typ aufweisen und durch Deletion und/oder Substitution erhalten werden, **dadurch gekennzeichnet, dass** sie mindestens 60 % Homologie zu der Sequenz ID 1 aufweisen.

2. EPIL/Placentin nach Anspruch 1 mit der Sequenz ID 1.

3. EPIL/Placentin oder Analoga von EPIL/Placentin nach einem der Ansprüche 1 bis 2, **dadurch gekennzeichnet, dass** das Protein glycosyliert ist.

4. DNA-Molekül, ausgewählt aus:
(a) der DNA-Sequenz mit der Sequenz ID 1,
(b) den DNA-Sequenzen, die unter Berücksichtigung der Degeneration des genetischen Codes für EPIL/Placentin oder dessen Analoga nach Anspruch 1 oder 2 codieren.

5. DNA-Molekül mit der Sequenz ID 1.

6. Expressionsvektor, der EPIL/Placentin oder ein Analogon von EPIL/Placentin nach einem der Ansprüche 1 bis 2 unter der Kontrolle von Elementen exprimiert, welche dessen Expression in einer Wirtszelle sicherstellen können.

7. Transformierte Wirtszelle, **dadurch gekennzeichnet, dass** sie einen selbstreplizierenden Vektor umfasst, der EPIL/Placentin oder ein Analogon von EPIL/Placentin nach einem der Ansprüche 1 bis 2 exprimiert.

8. Zelle nach Anspruch 7, **dadurch gekennzeichnet, dass** es sich um eine eukaryotische Zelle handelt.

9. Zelle nach Anspruch 7, **dadurch gekennzeichnet, dass** es sich um ein Bakterium handelt.

10. Verfahren zur Herstellung von EPIL/Placentin oder einem Analogon von EPIL/Placentin nach einem der Ansprüche 1 bis 2, **gekennzeichnet durch** das In-Kultur-Geben von Zellen nach einem der Ansprüche 7 bis 9, aus denen man direkt oder ausgehend vom Kulturmedium EPIL/Placentin oder dessen Analoga extrahiert.

11. EPIL/Placentin oder Analogon von EPIL/Placentin, erhalten durch das Verfahren nach Anspruch 10.

12. Pharmazeutische Zusammensetzung, **dadurch gekennzeichnet, dass** sie EPIL/Placentin oder ein Analogon von EPIL/Placentin nach einem der Ansprüche 1 bis 3 enthält.

13. Monoklonale Antikörper, gerichtet gegen EPIL/Placentin oder ein Analogon von EPIL/Placentin nach einem der Ansprüche 1 bis 3.

14. In-vitro-Diagnoseverfahren, das EPIL/Placentin oder ein Analogon von EPIL/Placentin nach einem der Ansprüche 1 bis 3 oder einen monoklonalen Antikörper nach Anspruch 13 verwendet.

## Claims

1. EPIL/placentin, a protein having sequence ID 1, and its analogues exhibiting EPIL/placentin-type activity and obtained by deletion and/or substitution, **characterized in that** they exhibit at least 60% homology with sequence ID 1.

2. EPIL/placentin according to Claim 1, having sequence ID 1.

3. EPIL/placentin or EPIL/placentin analogues according to either of Claims 1 and 2, **characterized in that** the protein is glycosylated.

4. A DNA molecule chosen from:
(a) the DNA sequence having the sequence ID 1,
(b) the DNA sequences that, taking into account the degeneracy of the genetic code, encode EPIL/placentin or its analogues according to Claim 1 or 2.

5. A DNA molecule having sequence ID 1.

6. Expression vector expressing EPIL/placentin or an EPIL/placentin analogue according to either of Claims 1 and 2, under the control of elements capable of providing its expression in a host cell.

7. Transformed host cell, **characterized in that** it contains a self-replicating vector expressing EPIL/placentin or an EPIL/placentin analogue according to either of Claims 1 and 2.

8. Cell according to Claim 7, **characterized in that** it is a eukaryotic cell.

9. Cell according to Claim 7, **characterized in that** it is a bacterium.

10. Method for preparing EPIL/placentin or an EPIL/placentin analogue according to either of Claims 1 and 2, **characterized by** the culturing of cells according to one of Claims 7 to 9, from which the EPIL/placentin or its analogues will be extracted directly or using the culture medium.

11. EPIL/placentin or EPIL/placentin analogue obtained by means of the method according to Claim 10.

12. Pharmaceutical composition, **characterized in that** it contains an EPIL/placentin or an EPIL/placentin analogue according to one of Claims 1 to 3.

13. Monoclonal antibodies directed against EPIL/placentin or an EPIL/placentin analogue according to one of Claims 1 to 3.

14. Method of diagnosing in vitro using EPIL/placentin or an EPIL/placentin analogue according to one of Claims 1 to 3 or a monoclonal antibody according to Claim 13.
